# EUROPEAN PATENT APPLICATION

(11) **EP 1 491 145 A1**
(43) Date of publication of application: **29.12.2004**
(21) Application number: 03745901.3
(22) Date of filing: 03.04.2003
(51) Int. Cl.: A61B 6/14

(54) **TOMOGRAPH**

(30) Priority: 04.04.2002 JP 2002103124
(71) Applicant: Hitachi Medical Corporation, Tokyo 100-0047 (JP)
(72) Inventor: MIYANO, Iwao, Nagareyama-shi, Chiba 270-0121 (JP)
(74) Representative: Strehl Schübel-Hopf & Partner
(86) International application number: PCT/JP2003/004286
(87) International publication number: WO 2003/084406

(57) **Abstract**

An X-ray CT apparatus according to the present invention is designed to greatly shorten the time taken for image processings in irradiating a cone beam X-ray to the whole jaw including a dental arc and to a local region such as a region around a tooth and a jaw joint and obtaining a panoramic image showing conditions of a dental row, teethridge, and tissue and bone. The imaging apparatus includes a reverse L-shaped fixing column, a rotative arm suspended at the end thereof, and X-ray generating device and a two-dimensional X-ray detecting device oppositely fixed to the ends of rotative arm respectively. The rotative arm is horizontally supported in the rotatable state via a dual rotation system with respect to the column. An object to be examined sits on a chair which is movable upward and downward, and an examining region is adjusted to a height of imaging center of the imaging apparatus. The object's head is fixed by a fine-adjustable head holder. Imaging is performed so that a distance between rotation centers of the two rotation systems substantially coincides with the object's dental arc.

## Description

### Technical Field

The present invention relates to an X-ray CT apparatus for obtaining a tomographic image and the like by irradiating an X-ray to a portion of an object to be examined and processing a projection image of the region, more particularly to an X-ray CT apparatus which can obtain an arbitrary CT image and a panoramic image of the region by irradiating a cone beam X-ray and is suitable for imaging in dental treatment.

### Background of the Invention

In current dental treatment, general imaging in which a film is attached in the back of teeth to perform X-ray imaging, panoramic imaging in which an X-ray tube and a film are simultaneously revolved, cephalometric imaging in which an X-ray tube is greatly detached from a film during imaging, and the like are performed. The X-ray panoramic imaging for dentistry is an imaging method in which curved cross sections are sequentially imaged along a tooth row and the tomographic images are spread out and displayed as one panoramic image showing conditions of the tooth row and tissue and bone around it.

In a conventional panoramic imaging apparatus, a rotative arm mounting an X-ray generating device and a two-dimensional X-ray detecting device facing to each other and interposing the object is supported by, e.g. a back-forth / left-right movable unit and rotative unit, which is designed to move around the object along a complicated orbit simulating the shape of a dental arc therebetween. An example of tomograph for dentistry of this kind is disclosed in Japanese Unexamined Patent Publication No.Hei06-78919.

Also, as a dental X-ray imaging apparatus, an X-ray CT apparatus which can obtain a horizontal tomographic image of tooth other than the panoramic image is proposed. The apparatuses of this kind include, for example, a medical X-ray tomograph mentioned in Japanese Unexamined Patent Publication No.Hei 09-122118 (the first conventional technique) and a panoramic X-ray imaging apparatus mentioned in Japanese Unexamined Patent Publication No.Hei 11-318886 (the second conventional technique). A known example of general medical CT apparatus using a cone beam X-ray is mentioned in Japanese Unexamined Patent Publication No.Hei 10-192267 (the third conventional technique). According to this apparatus, a tomographic image covering a wide region of the object can be obtained so as to be applicable to imaging on jaw including the dental arc.

Meanwhile, a technique involved with an X-ray CT imaging method and apparatus for irradiating a cone beam X-ray only to one portion of the object and obtaining an arbitrary tomographic image or a three-dimensional image of the portion is mentioned as a local irradiation X-ray CT apparatus in Japanese Unexamined Patent Publication No.2000-139902 (the fourth conventional technique). Specially, in dental application, a cone beam X-ray is irradiated with rotation not to the whole jaw including the dental arc but only to a local region limited around a tooth and jaw joint to thereby reduce an exposure dose and a CT image and a three-dimensional image of high resolution are provided.

It is known that in any of the above conventional techniques, it takes very long time (20 minutes to about one hour) for image calculation processing after imaging until an image is presented on an image display device.

The present invention is done in consideration of the above described points, and its object is to provide an X-ray CT apparatus which can greatly shorten the time taken for image processing when a panoramic image showing conditions of a tooth row, teethridge, and tissue and bone around them is obtained.

### Summary of the Invention

To achieve the above object, an X-ray CT apparatus according to the present invention includes X-ray generating means for generating an X-ray, X-ray detecting means for two-dimensionally detecting X-ray dose which is transmitted through an object to be examined, holding means for holding the X-ray generating means and the X-ray detecting means so that the object is located therebetween, first rotation driving means for driving the holding means to rotate around the object, containing means attached to the holding means for containing the first rotation driving means, image processing means for producing an image involved with the object on the basis of the X-ray dose detected by the X-ray detecting means, and image display means for displaying an image created by the image processing means, further including second rotation driving means for rotating as one body the holding means and the containing means, wherein the second rotation driving means contained in the containing means in parallel with a rotation center of the first rotation driving means and in different relation of rotation center position that of the first rotation driving means, and drive control means for controlling driving of the first rotation driving means in a first imaging mode and separately driving the second rotation driving means and the second driving means in a second imaging mode.

The first rotation driving means is designed to rotate the holding means for holding the X-ray generating means and the X-ray detecting means arranged opposite to each other with respect to the object. An X-ray is irradiated while the X-ray generating means and the X-ray detecting means rotate around a local region in the vicinity of rotation center of the first rotation driving means. By the second rotation driving means is driven and rotate as one body the holding means and the first rotation driving means, the rotation center of the first rotation driving means is revolved on a predetermined circumference. Then in the first imaging mode (CT imaging), a location of a local X-ray irradiating region is determined by moving the rotation center of the first rotation driving means on approximate circumference of dental arc by the second rotation driving means. In the second imaging mode (panoramic imaging), the rotation center of the first rotation driving means moves along the approximate circumference of the shape of dental arc, while a rotation angle, i.e. an imaging direction can be properly adjusted so that an irradiating direction is substantially perpendicular to the dental arc.

Because a most suitable panoramic image can be thus obtained by using a simple mechanical means, a complicated image calculating processing becomes unnecessary, whereby the time necessary for image processings in obtaining a panoramic image can be greatly shortened. When a distance between the X-ray generation source and the object varies due to the difference between the shape of circumference and that of actual dental arc, and so an expansion ratio of fluoroscopic image varies depending on a tooth position, a proper panoramic image can be obtained by correcting the variation in the image calculating processings on each of acquired local data in synchronism with the rotation angle of the rotation mechanism moving along the approximate circumference of the dental arc, and reconstructing the whole image.

### Brief Description of the Drawings

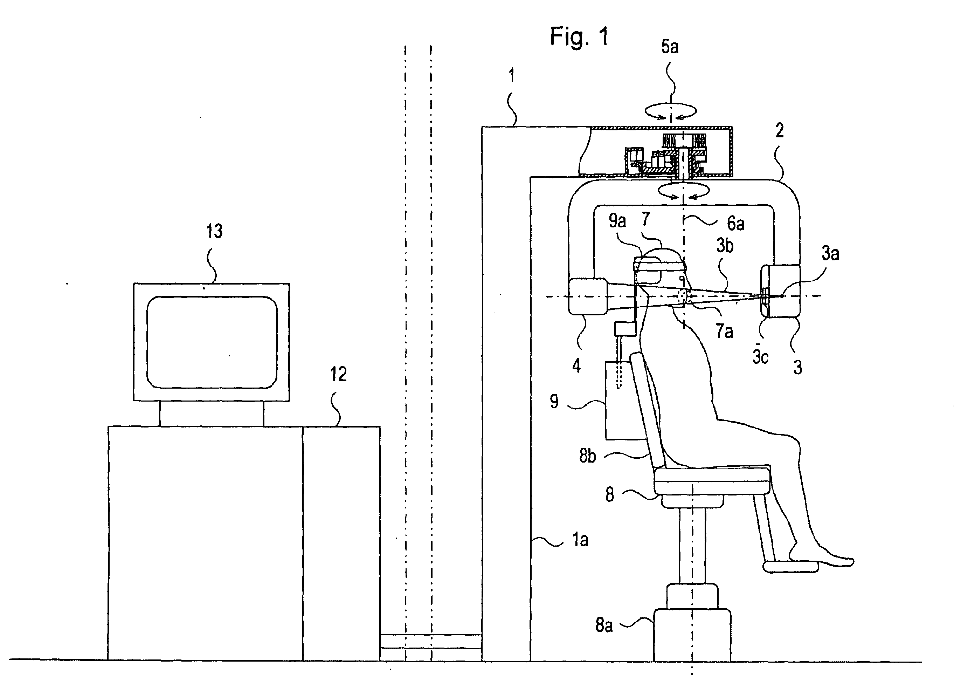

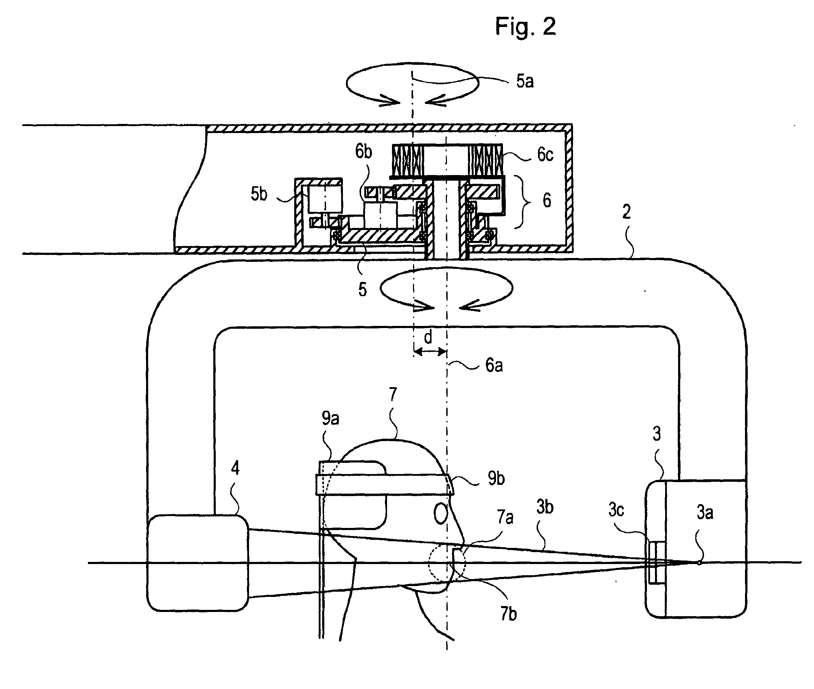

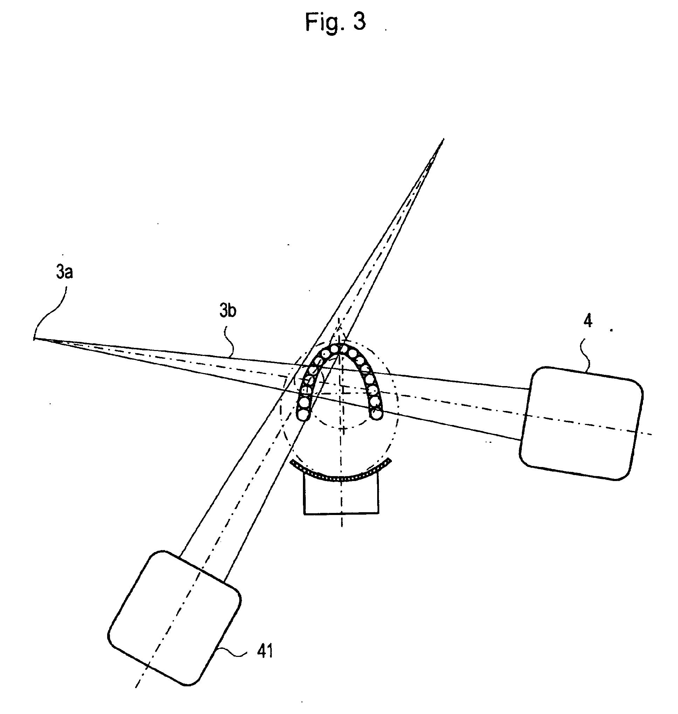

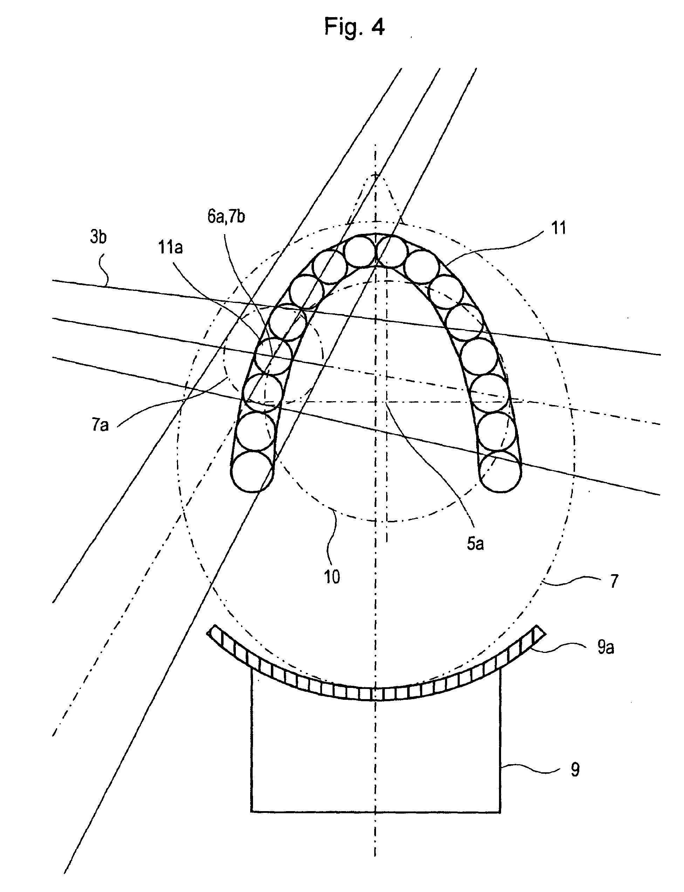

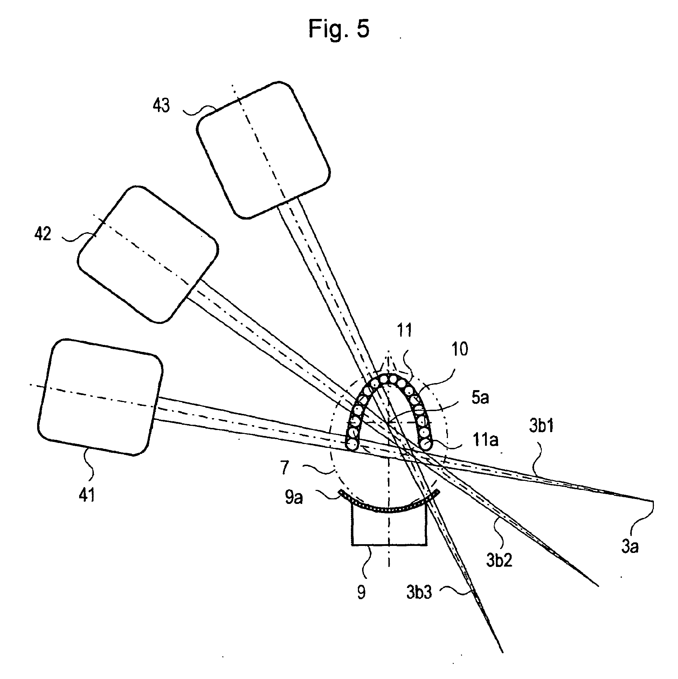

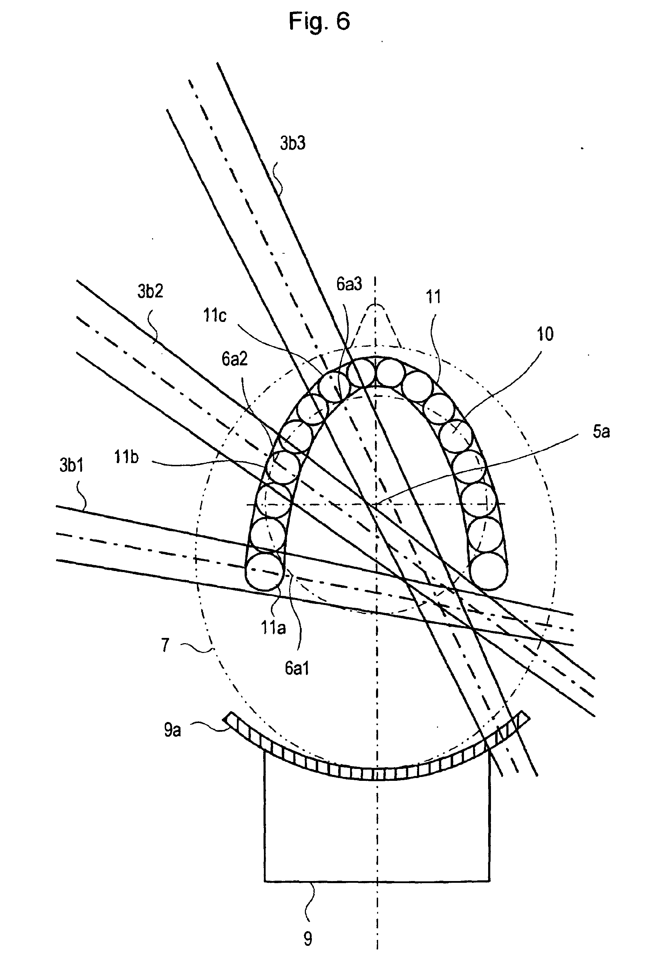

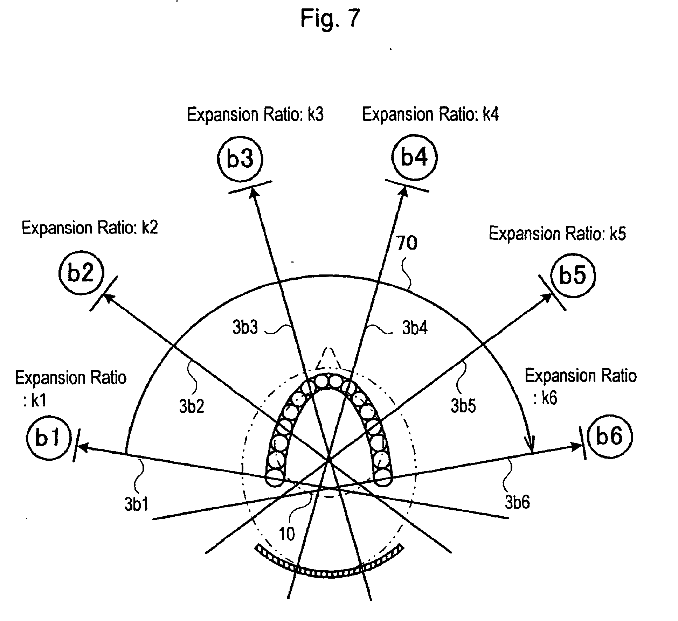

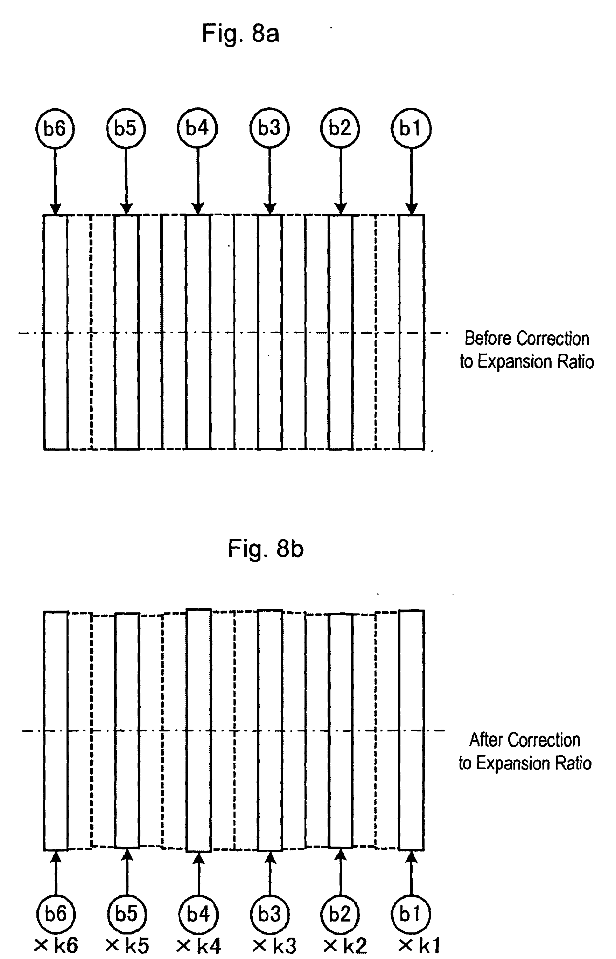

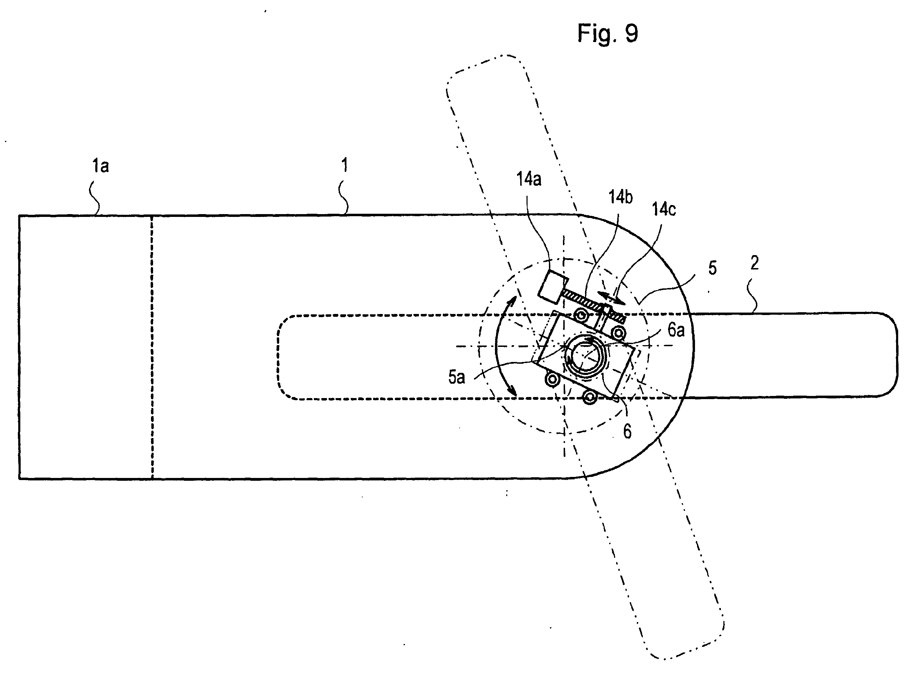

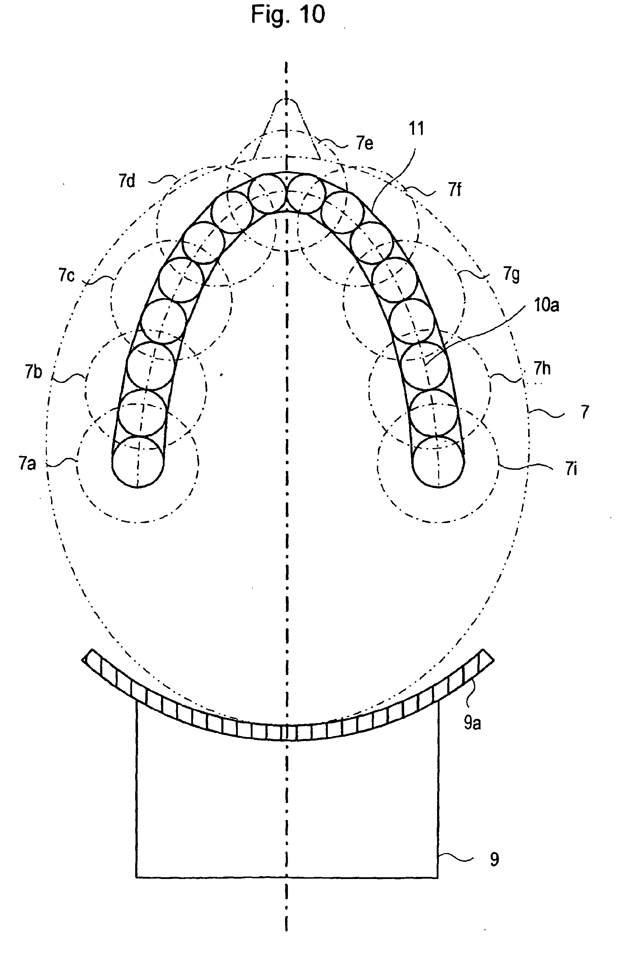

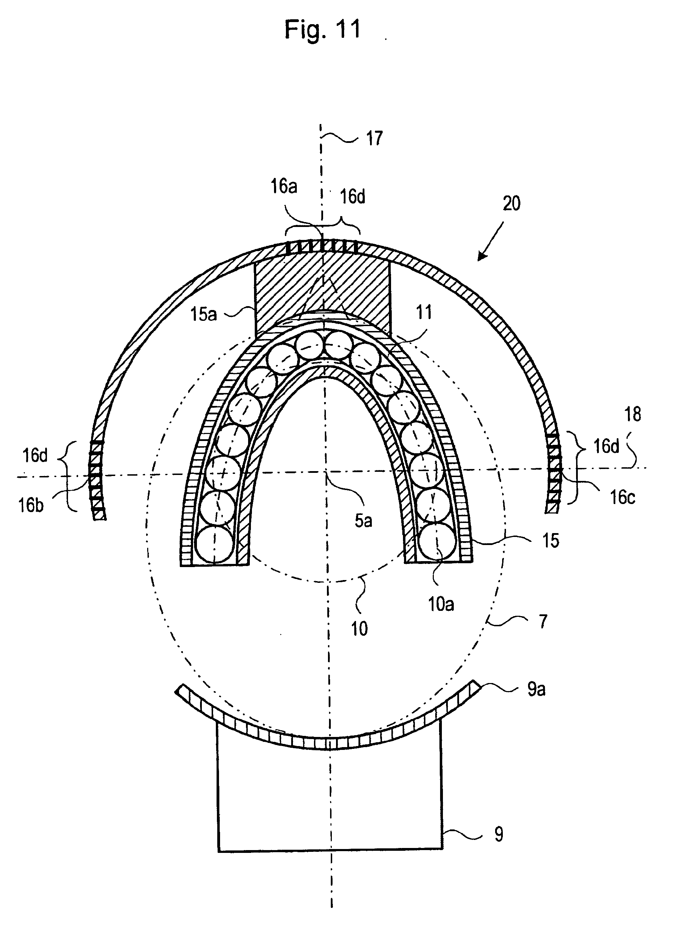

Fig.1 is a side view showing a schematic structure of an X-ray CT apparatus according to the present invention and also showing a cross sectional structure of one portion. Fig.2 is a partial enlarged view of the cross sectional structure of one portion shown in Fig.1 for easy understanding. Fig.3 is a diagram showing a procedure of positioning in a case where imaging is performed by the X-ray CT apparatus according to the embodiment of Fig.1. Fig.4 is a partial enlarged view of Fig.3. Fig.5 is a diagram showing an operation in a case where panoramic imaging is performed by the X-ray CT apparatus according to the embodiment of Fig.1. Fig.6 is a partial enlarged view of Fig.5. Fig.7 is a diagram showing differences between a rotation center of first rotation system 6 and centers of teeth in a case where a rotation angle of the second rotation system is changed at every irradiation. Fig.8 is a diagram showing the state of before and after correction of an expansion ratio of fluoroscopic images taken by each cone beam X-ray of Fig.7. Fig.9 is a top view of a variation of a rotation mechanism of the second rotation system in the X-ray CT apparatus according the present invention. Fig.10 is a diagram showing a rotation radius drawn by the X-ray CT apparatus of Fig.9. Fig.11 is a schematic structure of a positioning device used in the X-ray CT apparatus according to this embodiment.

### Best Mode for Carrying Out the Invention

Hereinafter, a preferable embodiment of an X-ray CT apparatus according to the present invention will be described with reference to the accompanying drawings. Fig.1 is a side view showing a schematic structure of the X-ray CT apparatus according to the present invention, which also shows the structure of a cross section of a region. Fig.2 is a partial enlarged view showing a partial cross sectional structure of Fig.1 for easy understanding.

This X-ray CT apparatus includes fixing column 1, rotative arm 2, X-ray generating device 3, two-dimensional X-ray detecting device 4, first rotation system 6, second rotation system 5, chair 8, and head holder 9. Fixing column 1 being in a reversed L-shape, is supported by column 1a and includes second rotation system 5 and first rotation system 6 on its ends. Rotative arm 2 is suspended from the end of fixing column 1. First rotation system 6 holds rotative arm 2 in the suspended state and rotates arm 2 at a predetermined speed around a rotation center of rotation shaft 6a at the end of fixing column 1. Second rotation system 5 is designed to rotate the whole first rotation system 6 at a predetermined speed around rotation shaft 5a. That is, positional relationship between second rotation system 5 and first rotation system 6 is that they are arranged in parallel and have different rotational center, and rotation systems are contained in a common containing unit. Second rotation system 5 and first rotation system 6 will be described later in detail.

Although second rotation system 5, first rotation system 6, and rotative arm 2 attached thereto are arranged over the head of object 7 in the above example, they may be arranged in the direction of the feet of object 7. By doing so, when object 7 sits on the chair in accordance with the imaging position of the X-ray CT apparatus, object 7 needs not worry about collision with the rotation system including rotative arm 2.

X-ray generating device 3 is designed to generate an X-ray, which is located on one end of rotative arm 2. X-ray generating device 3 includes collimator device 3c for narrowing down X-ray 3b irradiated from X-ray generation source 3a inside the X-ray generating device 3 into a cone-shaped beam. Two-dimensional X-ray detecting device 4 arranged opposite to X-ray generating device 3 is designed to two-dimensionally detect an X-ray dose transmitted through the object and installed in the other end of rotative arm 2. That is, X-ray generating device 3 and two-dimensional X-ray detecting device 4 are arranged opposite to each other through rotative arm 2. Rotative arm 2 is driven by first rotation system 6 to rotate by approximately 405° around a rotation center being the tip of fixing column 1. Although the imaging range is 360°, the rotation range is wider than it by 45° because imaging is started when the rotation speed becomes constant. The rotation range is widely set for accelerating the rotation until the rotation of first rotation system 6 becomes constant and decelerating the rotation until first rotation system 6 stops after imaging, and so it is not limited to 45°. After imaging is started, X-ray generating device 3 irradiates an X-ray in pulse form in synchronism with image acquisition, and X-ray exposure to the object is thus reduced. The timing thereof is controlled by a positional detection encoder built into first rotation system 6. Inside column 1a of fixing column 1, a control system of imaging apparatus is installed.

Image data acquired by two-dimensional X-ray detecting device 4 are sent to image processing device 12. Image processing unit 12 is installed in an operation room distant from an imaging room in which the X-ray CT apparatus is installed. Image processing device 12 performs a calculation processing on received image data, reconstructs a two-dimensional tomographic image, a CT image, or a three-dimensional image, and presents the image on image display device 13. Image display device 13 includes an input device such as a keyboard and a mouse (not shown) and works so that image processing device 12 functions as a computer device. Because conditions of image reconstruction can be input from this input device, it is possible to input which of the two-dimensional tomographic image, CT image, three-dimensional image, and panoramic image as a subject of the reconstruction.

Rotative arm 2 is supported rotatably and horizontally relative to fixing column 1. In this embodiment, rotative arm 2 is constructed to be an up-down dual structure including second rotation system 5 and first rotation system 6. Second and first rotation systems 5 and 6 include a rotation supporting mechanism using a bearing, a driving mechanism and a position detecting mechanism rotated by the combination of a servomotor and a gear, and a cable processing mechanism of rotation unit. Rotation center 5a of second rotation system 5 is fixed to fixing column 1 and rotation center 6a of first rotation system 6 is fixed to rotative arm 2. Rotation centers 5a and 6a are detached from each other by a fixed distance d. Rotation center 6a of first rotation system 6 is rotated by driving device 5b of second rotation system 5 around a rotation axis being rotation center 5a of second rotation system 5. And, driving device 5b (including the position detecting device) of second rotation system 5 and a cable processing mechanism (concrete structure being not shown) are contained inside fixing column 1. Driving device 6b of first rotation system (including the position detecting device) and cable processing mechanism 6c are contained in the upper part of second rotation system 5. Distance d between two rotation centers 5a and 6a substantially corresponds to the size of the dental arc of object 7, e.g. a diameter of around 70 to 100mm. First rotation system 6 has to be rotate by 360° or more (around 405°) to acquire CT image data. On the other hand, because second rotation system 5 is provided for the purpose of making the rotation similar to the dental arc, it is sufficient to rotate ±120° to the maximum.

Further, because there are individual differences in remaining teeth and the like of object 7, the maximum rotation angle of second rotation system 5 is not limited to ±120°, and it can be arbitrarily set using an input device.

The cable processing mechanism of second rotation system 5 and first rotation system 6 is commonly provided by using a guiding rail along the movement of cable caused by the rotations. Accordingly, a plurality of cable processing mechanisms are unnecessary and an installation space of the mechanism can be miniaturized.

Meanwhile, object 7 sits on chair 8 which can be moved upward and downward. The position of examining region 7a of object 7 is determined to a height of an imaging center of the imaging apparatus. An angle of chair back 8b of chair 8 can be adjusted at an arbitrary angle. The position of object 7 in a back-front direction is substantially adjusted by combining the angle adjustment to chair back 8b and the position adjustment done by up-down movement mechanism 8a. Head holder 9 is provided in the rear of chair back 8b and adjustable upward and downward, backward and forward, and left and right in accordance with a seated height of object 7 and the examining region so as to fix the head of object 7 to a desirable position of chair 8. The head of object 7 is moved to the desirable position by an operator and fixed by head band 9b or the like. A center of examining region 7a of object 7 can be adjusted to the rotation center 6a of rotative arm 2 (rotation center of first rotation system 6 mentioned above) by the operator.

Chair 8 is moved upward and downward by up-down movement mechanism. If head holder 9 is attached to angle-adjustable chair back 8b, it is not necessary to use the dedicated chair according to the embodiment.

That is, if the specifications of up-down stroke and the like are fulfilled, a chair used in, e.g. the hairdresser may be utilized. Further, a therapeutic chair used in the otorhinolaryngology also may be utilized.

Further, in Fig.1, a state that the back of object 7 is directed back to column 1a perpendicular to the floor on which fixing column 1 is installed. However, because the imaging apparatus is separated from the chair in this embodiment, the angle of the object's position relative to column 1a is not limited as long as a rotation range resides in CT imaging. For example, imaging also can be performed while object 7 is put to face column 1a. In this case, by setting a projector of an optical marker for positioning to the column, the optical marker can be projected from a direction opposite to object 7, whereby positioning becomes easy. Alternatively, the setting direction of column 1a and chair 8 can be freely set in relation to a layout of the imaging room where the apparatus is installed.

Fig.3 is a diagram showing the procedure of positioning when imaging is performed by the X-ray CT apparatus according to this embodiment, and Fig.4 is a partial enlarged view of Fig.3. When CT imaging is performed locally on one or two teeth only, second rotation system 5 is revolved so as to match center 7b of the region where tooth 11 a to be imaged is located with rotation center 6a of first rotation system 6 (rotative arm) of the X-ray CT apparatus. However, circle 10, the rotation radius of which is constant distance d between the rotation center 5a of second rotation system 5 and rotation center 6a of first rotation system 6, is made substantially to coincide with the shape and size of dental arc 11. Accordingly, circle 10 does not always coincide with dental arc 11 depending on the position of tooth. That is, center 7b of examining region cannot be matched with center 6a of the rotative arm only by making object 7 sit on the center in the left-right direction of chair 8.

If imaging region 7a has enough size to include several teeth so that the examining region is located in the vicinity of the center of imaging range 7a, imaging is sufficiently performed even when center 7b of the examining region is detached a little from center 6a of the rotative arm. However, when the imaging region is limited to one or two teeth because of limitation of a detectable size of two-dimensional X-ray detecting device 4, or when the shape and size of dental arc 11 is individually different as between an adult and a child so that an apparent differences occur between the shape and size of circle 10 having a rotation radius being distance d between rotation centers 5a and 6b of second and rotation system 5 and first rotation system 6 in the X-ray CT apparatus according to this embodiment and those of the object's dental arc, and resultingly tooth 11a being the examining region is possibly out of imaging region 7a, it is necessary to match center 7b of the examining region and rotation center 6a of the rotative arm as accurately as possible.

According to this embodiment, the object's position in the back-front direction is adjusted by properly combining the angle adjustment to chair back 8b of chair 8 and the up-down position adjustment to up-down movement mechanism 8a to substantially match center 7b of examining region 11a with rotation center 6a of first rotation system 6 (rotative arm 2). After fixing the head to head holder 9a of chair 8, the head position is fine adjusted by using the back-front and left-right movement mechanism of head holder 9. In this manner, center 7b of examining region 11a can be completely matched with center 6a of first rotation system 6 (rotative arm 2).

In the above-described positioning procedure, the positioning is generally performed with reference to a linear optical marker projected to the body surface of object 7 in the state that the object's mouth cavity is closed. Therefore, in some cases, it is difficult to check from outside whether or not the position of tooth imaged completely coincides with the center of the imaging range.

In this case, a more accurate method of positioning is applicable, in which positioning is done using the optical marker from outside as described above, the direction of rotative arm 2 of the imaging apparatus is then changed, and X-ray fluoroscopic imaging is performed from two orthogonal directions, thereby the position of object 7 is remotely and finely adjusted while the position of teeth is visually checked on a fluoroscopic image. In this case, the position adjustment can be done more precisely and accurately by directly finely adjusting the position of head of object 7 by remote controlling head holder 9, rather than by performing fine adjustment while moving object 7. Further, from the viewpoint of safety, too, a moving distance of object 7 is desirably kept to the minimum in order to prevent object 7 from touching the X-ray CT apparatus, and to reduce external force applied to object 7 and a moving distance of object 7. In the case of the X-ray CT apparatus according to this embodiment, it is characteristic that after adjusting the rough position in the apparatus, head holder of chair 8 is separately finely adjusted. According to this embodiment, the range of fine adjustment to the head is around ±15mm at most. Accordingly, a burden on the object caused by movement of head holder 9 is much smaller in comparison with the case that the position adjustment is done by moving the entire object (±50mm to the maximum). Furthermore, it is possible to improve accuracy of positioning and to shorten the time taken therefor.

Further, other than the positioning using the rotation mechanism, it is also applicable to use the second rotation system only in panoramic imaging, and positioning of object 7 is done by combining up-down movement of the chair and up-down, back-forth, and left-right movement of head holder 9.

Since rotative arm 2 rotates around head holder 9 to irradiate a cone beam X-ray, head holder 9a to which cone beam X-ray 3b is irradiated is desirably made of a permeable material to radioactive rays and enough strength to hold and fix the head, such as carbon fiber, so that head holder 9a does not become an obstacle to image data acquisition by reason that an X-ray is absorbed.

An advantage of using head holder 9 is that since the back of the head of object 7 is fixed by head holder 9a, safety in a region where object 7 cannot visually check (i.e. back of the head) by himself/herself is ensured if it is ensured in the apparatus that head holder 9a does not touch the main body of the X-ray CT apparatus during rotation of rotative arm 2.

Although only head holder 9 is used as means for fixing and positioning object 7 in this embodiment, the fixing and positioning means is not limited to head holder 9. It is possible to utilize a combination of a chin rest and an ear rod, or a fixing device using a dental articulation model produced in accordance with a denture model of each object in combination with the head holder. If those devices are constructed so that it can be fine adjusted back and forth, and left and right, the similar positioning function can be realized by fine adjusting their position.

In the state that the position of object 7 is fixed as described above, CT imaging is performed by revolving rotative arm 2 while cone beam X-ray 3b is irradiated from X-ray generating device 3. In accordance with the rotation angle of rotative arm 2, two-dimensional X-ray detecting device 4 mounted opposite to X-ray generating device 3 (not shown) to the other end of rotative arm 2 is rotated from the position of two-dimensional X-ray detecting device 4 to that of two-dimensional X-ray detecting device 41. Eventually, fluoroscopic image data over 360° of diagnostic region 11a are acquired. The acquired image data are subjected to calculation processing at image processing device 12, a two-dimensional tomographic image or a three-dimensional image is reconstructed, and the image is displayed on image display device 13.

The execution of the above-described imaging process can be outlined in the following order of (1) to (5):
(1) An imaging region of object 7 is substantially positioned by rotating second rotation system 5.
(2) The position of object 7 is fixed by finely adjusting head holder 9 of object 8.
(3) If necessary, X-ray fluoroscopic imaging is performed from two orthogonal directions and the object's position is finely adjusted while the position of teeth is visually checked on the fluoroscopic image.
(4) CT image data are acquired by rotating first rotation system 6 (rotative arm 2) while cone beam X-ray 3b is irradiated.
(5) The acquired image data are subjected to calculation processing in image processing device 12 to reconstruct a two-dimensional tomographic image or a three-dimensional image, and the image is displayed on image display device 13.

Fig.5 is a diagram showing the operation when panoramic imaging is performed by the X-ray CT apparatus according to this embodiment, and Fig.6 is a partial enlarged view thereof. First, the center in the left-right direction of object 7 is in agreement with rotation center 5a of second rotation system 5. If the center in the left-right direction of chair 8 is preset to be just below the rotation center 5a of second rotation system 5, position adjustment to object 7 in the left-right direction is scarcely necessary except for fine adjustment of head holder 9. However, it is here postulated that dental arc 11 of object 7 is symmetrical with respect to the center of object 7 in the left-right direction. Subsequently, the position of object 7 in the back-front direction is properly adjusted combining the angle adjustment to chair back 8b of chair 8 and the position adjustment to up-down movement mechanism 8a in the up-down direction so as to substantially match the shape and size of dental arc 11 with circle 10, the rotation radius of which is distance d between rotation center 5a of second rotation system 5 and rotation center 6a of first rotation system 6. After fixing the rear of the head to head holder 9, the position thereof is finely adjusted by a back-front and left-right movement mechanism of head holder 9. In this manner, the rotation center 6a of first rotation system 6 is located on circle 10.

As described above, in the state that the position of object 7 is fixed, rotative arm 2 is revolved by first rotation system 6 in accordance with the rotation angle of second rotation system 5 while second rotation system 5 is revolved. A tomogram of dental arc 11 is obtained by irradiating cone beam X-ray 3b in a direction perpendicular to each tooth of dental arc 11 which does not interfere an opposite tooth of dental arc 11. Because of differences in the shape and size between actual dental arc 11 and circle 10 having a rotation radius of distance d between rotation center 5a of second rotation system 5 and rotation center 6a of first rotation system 6, the distance between X-ray generation source 3a and object 7 varies depending on the position of tooth. As a result, an expansion ratio and a density of fluoroscopic image projected to two-dimensional X-ray detector 4 vary. That is, when tooth 11a is imaged, although cone beam X-ray 3b1 irradiated from X-ray generator 3 is transmitted through the center of rotation center 6a1 of first rotation system 6 and tooth 1, there is a little distance between rotation center 6a1 of first rotation system 6 and the center of tooth 11b.

In a similar manner, when tooth 11 b is imaged, although cone beam X-ray 3b2 irradiated from the X-ray generator 3 is transmitted trough rotation center 6a2 of first rotation system 6 and the center of tooth 11b, there is a little distance between rotation center 6a2 of first rotation system 6 and the center of tooth 11b. When tooth 11c is imaged, although cone beam X-ray 3b3 irradiated the X-ray generator 3 is transmitted through rotation center 6a3 of first rotation system 6 and the center of tooth 11c, there is a little distance between rotation center 6a3 of first rotation system 6 and the center of tooth 11c.

Accordingly, after the differences therebetween are corrected in the image calculating processing in synchronism with the rotation angle of second rotation system 5, partial image data at each angle are joined to reconstruct a continuous image over the whole angle. Thus, an accurate panoramic image can be obtained. Fig.7 is a diagram showing differences between the rotation center of first rotation system 6 and the center of the respective teeth in the case that the rotation angle of second rotation system 5 is varied at each time. In accordance with a tooth to be imaged gradually shifted from the left end to the right end, the rotation center 6a of first rotation system 6 moves on circle 10. According thereto, the irradiation angle of cone beam X-ray 3b1 to 3b6 is gradually varied like arrow 70. By irradiating cone beam X-rays 3b1 to 3b6, fluoroscopic image b1 to b6 are obtained by two-dimensional X-ray detecting device 42. Because of the differences between the shape and size of the actual dental arc and those of circle 10 having a rotation radius being distance d between rotation center 5a of second rotation system 5 and rotation center 6a of first rotation system 6, difference occurs in the distance between X-ray generator 3a and object 7 depending on the position of tooth. Resultingly, difference occurs in the expansion ratio and density of the fluoroscopic image projected on two-dimensional X-ray detecting device 4.

Fig.8 is a diagram showing the state of fluoroscopic images b1 to b6 obtained by cone beam X-rays 3b1 to 3b6 before and after correction of expansion ratio. As shown in Fig.8(A), the each of fluoroscopic images b1 to b5 before the correction of the expansion ratio substantially has the same size. By multiplying those fluoroscopic images b1 to b6 before correction by expansion ratios k1 to k6 respectively in accordance with the difference between the rotation center of first rotation center 6 (point on circle 10) and the center of each tooth, the sizes of fluoroscopic images b1 to b6 can be corrected as shown in Fig.8(B). Then, a panoramic image is reconstructed on the basis of the size-corrected image. Meanwhile, although the density of the image is not shown in the figure, it is needless to say that the density is also corrected.

In this correction processing, the amount of information to be dealt with is small, and so the correction processing itself is simple.

Further, if two kinds of dental arcs of standard size are prepared respectively for adults and children as the shape and size of dental arc 11 to be the reference in correction, the correction can be automatically done with reference thereto and only two kinds of tables of correction coefficient used on the software are necessary. Accordingly, it is possible to reduce memory capacity installed in image processing device 12 and shorten the time taken for image processing. If the shape and size of dental arc 11 customized for each individual can be produced other than those of the reference size on the software, it is needless to say that more accurate correction can be done.

The execution of the above panoramic imaging procedure is outlined in the following order of (1) to (6):
(1) By combining the angle adjustment of chair back of 8b of chair 8 and the up-down position adjustment of up-down movement mechanism 8a, the imaging region of object 7 is positioned so that trajectory 10 (circular trajectory) drawn by rotation center 6a of rotative arm 2 (first rotation system 6) substantially coincides with dental arc 11.
(2) Head holder 9 of chair 8 is fine adjusted to fix the position of object 7.
(3) Second rotation system 5 is revolved to adjust an end of dental arc 11 (back tooth) to an irradiation starting position. At the same time, the rotation angle of rotative arm 2 is adjusted in a direction perpendicular to dental arc 11 which does not interfere with an opposite tooth of dental arc 11, and irradiation of cone beam X-ray 3b is started.
(4) While second rotation system 5 is revolved along dental arc 11, the rotation angle of rotative arm 2 is adjusted in a direction perpendicular to dental arc 11 which does not interfere with each of opposite teeth of dental arc 11, cone beam X-ray 3b is sequentially irradiated, and thus partial fluoroscopic image data are acquired at each rotation angle of second rotation system 5.
(5) Imaging procedure is completed when the position of data acquisition of partial fluoroscopic image reaches the other end of the dental arc (back tooth opposite to the starting position).
(6) After the expansion ratio and density of partial fluoroscopic image data acquired at each rotation angle of second rotation system 5 are corrected in image processing device 12 in synchronism with each rotation angle of second rotation system 5, and a continuous panoramic image of dental arc 11 over the whole angle is reconstructed and presented on image display device 13.

Fig.9 is a diagram showing a variation of the rotation mechanism of the second rotation system of the X-ray CT apparatus according to the present invention, being a top view of the X-ray CT apparatus of Fig.1. Fig.10 is a diagram showing a rotation radius drawn by this X-ray CT apparatus, being an enlarged view corresponding to Fig.4. Although distance (rotation radius) d between rotation center 5a of second rotation system 5 and rotation center 6a of first rotation system 6 is fixed in the X-ray CT apparatus shown in Fig.1, in the X-ray CT apparatus in Fig.9 rotation radius d can be freely changed, and rotation center 6a of first rotation system 6 moves on a complicated trajectory along dental arc 11. A linear driving system includes driving means 14a, such as a servomotor, mounted on second rotation system 5, and linear driving mechanism 14b. such as a feed screw and a lack and pinion, driven by driving means 14a. In the linear driving system, rotation center 6a of first rotation system 6 is moved in the direction of arrow 14c, and thus distance (rotation radius) d between rotation center 5a of second rotation system 5 and rotation system 6a of first rotation system 6 is moved to a desired position. In this manner, by the linear driving system correcting the position of rotation center 6a of first rotation system 6, a trajectory of rotation center 6a of first rotation system 6 is drawn on curved line 10a taken along dental arc 11 as shown in Fig.10. Accordingly, because a difference between rotation center 6a of first rotation system 6 and the center of teeth 11 does not occur, a correction processing done in the image calculating processing becomes unnecessary, and so the calculation time can be shortened.

The moving range of rotation center 6a of first rotation system 6 moved by the linear driving system is difference corresponding to a distance between the center of each tooth of dental arc 11 and rotation radius 10 in Fig.4, around ±15mm being enough. Further, if the moving distance is smaller, a load supporting/driving device can be miniaturized and bending and the like occurring due to the weight of apparatus can be reduced. Therefore, the position adjustment can be accurately done using a simple mechanism.

According to the X-ray CT apparatus in Fig.9, accurate positioning is easily performed in the apparatus, regardless of the shape and size of the dental arc of the object. If the apparatus is constructed so that this adjusting mechanism of the rotation radius can be remote controlled, fine adjustment to head holder 9 becomes unnecessary, whereby it is possible to greatly reduce a burden on object 7 and to simplify a mechanism for adjusting a position of chair 8 and fixing object 7. Further, image correction in regard to differences of expansion ratio and density of a fluoroscopic image obtained in the panoramic imaging also becomes unnecessary, whereby the time taken for the image calculation processing can be shortened.

Since it is not necessary to move the object in this X-ray CT apparatus, the following imaging method can be conducted. That is, as shown in Fig.10, X-ray CT imaging is sequentially and repeatedly executed plural times (nine times in Fig.10) on local regions 7a to 7i, each including two to three teeth, so as to cover the whole dental arc 11, and thus CT image data combining a plurality of local imaging regions 7a are acquired. In this manner, even when an X-ray detecting device of small FOV is used, CT image data for presenting the whole dental arc can be acquired.

The execution of the above imaging procedure on local region can be outlined in the following order of (1) to (7):
(1) An imaging region of object 7 is positioned and fixed so that a trajectory drawn by rotation center 6a of rotative arm 2 (dental arc-shaped trajectory) coincides with dental arc 11 of object 7.
(2) Rotation center 6a of rotative arm 2 is adjusted to the center of a back tooth located at one end of dental arc 11, i.e. local region 7a.
(3) cone beam X-ray 3b is irradiated while rotative arm 2 is revolved, and thus CT image data are acquired.
(4) Second rotation system 5 is revolved to adjust rotation center 6a of rotative arm 2 to a center of local region 7b adjacent to and partially overlapping with local region 7a, CT image data of which was acquired above.
(5) CT image acquisition and positioning are repeatedly done on local regions 7b to 7i along dental arc 11.
(6) The imaging procedure is completed when CT image data acquisition on the other end of dental arc 11(center of back tooth on the end opposite to the starting position, i.e. local region 7i) is finished.
(7) The CT image data acquired are subjected to the calculation processing in image processing device 12, and an image of the whole dental arc 11 is reconstructed and displayed on image display device 13.

According to this imaging method of local regions, an image of higher resolution can be obtained on an identical region. Further, it is also possible to extract fluoroscopic image data along the dental arc in a direction perpendicular to the tooth row from the above data and to reconstruct a panoramic image. Similarly, a tomographic image and a three-dimensional image of an arbitrary cross section can be reconstructed. Further, by limiting the X-ray irradiating region to a local region, an exposure dose of object 7 can be reduced. According to this embodiment, the exposure dose can be reduced as the number of times of CT imaging becomes smaller.

When the imaging method of local region is applied to the imaging method of Fig.3 to Fig.6, it is needless to say that similar imaging can be performed by sequentially repeating the local CT imaging while positioning the patient at every CT imaging on local region.

Meanwhile, although the X-ray CT apparatus according to this embodiment is suitable for dentistry, it is needless to say that this technique is not limited to dentistry and is applicable to general X-ray CT apparatus. For example, the method is applicable when an object to be examined is larger than an imaging range of the X-ray CT apparatus and in the case where sequential local CT imaging is performed on the whole object or in the case where panoramic imaging is performed from the inside of a cylindrical body simulating the shape of dental arc.

Fig.11 is a diagram showing a schematic structure of a positioning device used in the X-ray CT apparatus according to this embodiment. Positioning device 20 includes dental articulation unit 15 produced in accordance with a dental model of each object to be examined and flange 16 joined with dental articulation unit 15 via joint unit 15a. Flange 16 is made of a thin plate fixed onto a surface parallel to dental articulation unit 15. When object 7 puts on dental articulation unit 15 in the object's mouth, flange 16 is exposed outside the mouth of object 7 via joint unit 15a. On flange 16, line marks 16a to 16c are incused along orthogonal axes 17 and 18. Further, on the both sides of respective line marks 16a to 16c, scale marks 16d are incused at regular intervals around line mark 16a to 16c. Scale mark 16d gives an indication of distance when the object's position is shifted in CT imaging so as to match the center of the region imaged with rotation center 6a of rotative arm 2.

The X-ray CT apparatus is provided with a projector (not shown) for projecting an optical marker to a face of object 7 from three directions corresponding to line marks 16a to 16c of flange 16, which is positioned so that an intersecting point of those light axes passes through rotation center 5a of second rotation system 5. Then, by fine adjusting the position of object 7 wearing positioning device 20 so that line marks 16a to 16c coincide with the optical marker, the position of rotation center 5a of second rotation system 5 can be visually checked from the outside of mouth. Accordingly, accurate positioning can be realized only with the positioning based on the optical marker, without performing X-ray fluoroscopic imaging.

By changing the attaching position of joint unit 15a and flange 16, a positional relation between dental articulation unit 15 and rotation center 5a of second rotation system 5 can be changed. That is, adjustment can be done so that trajectory (circular trajectory) 10 drawn by rotation center 6a of rotative arm 2 substantially coincides with dental arc 11. In this adjustment, it is useful to prepare in advance a full-scale figure like one shown in Fig.11 for checking the state of correspondence of circular trajectory 10 and dental arc 11 and combine them while checking that the figure corresponds with the real thing. Further, this figure also may be used when it is checked that the intersecting point of light axes of the optical marker projector from three directions passes through rotation center 5a of second rotation system 5. Meanwhile, although the above is the description of the case that positioning device 20 is applied to the X-ray CT apparatus shown in Fig.3 to Fig.6, it also may be applied similarly to the X-ray CT apparatus shown in Figs.9 and 10 by using dental arc-shaped trajectory 10a instead of circular trajectory 10. Further, the above positioning processing may be automatically done by detecting positioning device 20 using a camera or the like and performing the image processing.

The execution of the imaging procedure using above positioning device 20 is outlined in the following order of (1) to (4):
(1) Positioning device 20 is worn by object 7 and dental articulation unit 15 is produced on the basis of a dental model of object 7.
(2) An attaching position of joint unit 15a and flange 16 is adjusted to connect and fix them so that trajectory (circular trajectory) 10 drawn by rotation center 6a of rotative arm 2 or dental arc-shaped trajectory 10a substantially coincides with dental arc 11.
(3) Dental articulation unit 15 of the device joined and fixed is put on to dental arc 11.
(4) Line marks 16a to 16c of flange 16 are made corresponding with the optical marker of the apparatus and the object is positioned.

According to the above-described embodiment, in the cone beam X-ray CT imaging apparatus for dentistry, positioning in local CT imaging can be easily done with a simple rotation mechanism, and the positioning mechanism can be simplified. Further, panoramic imaging also can be performed easily, and it is possible to greatly shorten the time for image calculation processing and to simplify the image processing device. Further, it is possible to perform local CT imaging and panoramic imaging without moving the object and to simplify the position adjustment of chair and an object fixing mechanism. Further, image correction in regard to differences in an expansion ratio and density of fluoroscopic image of imaging region becomes unnecessary. In an X-ray CT apparatus with a small FOV, too, CT image data of high resolution over the whole dental arc can be acquired by sequentially repeating the local CT imaging plural times. Further, a panoramic image of the whole dental arc, a tomographic image of an arbitrary cross section, and a three-dimensional image can be reconstructed from those image data.

Meanwhile, according to the above embodiment, rotative arm 2 is constructed as a single unit. However, it is also available to construct the rotative arm as a dual unit and relatively slide each of the unit so as to freely extend and contract its length in its radius direction and adjust the distance between X-ray generating device 3 and two-dimensional X-ray detecting device 4.

### Industrial Applicability

As described above, in an X-ray CT apparatus according to the present invention, when a cone beam X-ray is irradiated to the whole jaw including a dental arc and to a local region such as a tooth and around the jaw joint, and a panoramic image showing conditions of dental row, teethridge, and a tissue and a bone around them is obtained, the time taken for image processings can be extremely shortened.

## Claims

1. An X-ray CT apparatus comprising:
X-ray generating means for generating an X-ray;
X-ray detecting means arranged opposite to the X-ray generating means for two-dimensionally detecting an X-ray dose which is transmitted through an object to be examined;
holding means for holding the X-ray generating means and the X-ray detecting means so that the object is positioned therebetween;
first rotation driving means for driving the holding means to rotate along the circumference of the object;
containing means for containing the first rotation driving means attached to the holding means;
image processing means for producing an image of the object on the basis of the X-ray dose detected by the X-ray detecting means; and
image display means for displaying the image produced in the image processing means,
further comprising;
second rotation driving means to integrally rotate the holding means contained in the containing means and the containing means in a manner that the rotation center of the second rotation driving mean is in parallel with the rotation center of the first rotation driving means and is located at a different position from that of the first rotation driving means; and
drive control means for controlling the first rotation driving means in a first imaging mode and separately controlling the first rotation driving means and the second rotation driving means in a second imaging mode.

2. An X-ray CT apparatus according to claim 1, wherein the drive control means performs the control for execution of each of the first imaging mode and the second imaging mode.

3. An X-ray CT apparatus according to claim 1, wherein the image processing means reconstructs a two-dimensional tomographic image or a three-dimensional image of the object in the first imaging mode, and reconstructs a panoramic image of the object in the second imaging mode.

4. An X-ray CT apparatus according to claim 1, wherein each of the rotation center of the first rotation driving means and the second rotation driving means is arranged so that the distance therebetween is determined on the basis of a size of imaging region of the object.

5. An X-ray CT apparatus according to claim 1, wherein a rotation angle of the second rotation driving means is determined so that each of the holding means and the first rotation driving means is located in a predetermined imaging region of the object.

6. An X-ray CT apparatus according to claim 1, wherein in the case of executing the second imaging mode, differences in an expansion ratio of fluoroscopic image of the object, which occur due to differences in the distance between the circumference and each imaging region of the object, are corrected by an image calculating processing.

7. An X-ray CT apparatus according to claim 1, wherein the second rotation driving means is rotatable around a center of rotation axis of the rotative arm on a circumference simulating a shape of the imaging region of the object, positions the local X-ray irradiating region in the first imaging mode, and adjusts an imaging direction in the combination of the position of the irradiating region and a rotation angle of the rotative arm in the second imaging mode.

8. An X-ray CT apparatus according to claim 1, wherein the second rotation driving means is rotatable around a center of rotation axis of the rotative arm on a circumference simulating a shape of the imaging region of the object, and has a mechanism for varying a diameter of the circumference.

9. An X-ray CT apparatus according to claim 1, wherein by the locally repeating the first imaging mode plural times along the imaging region of the object with the second rotation driving means, image data in the first imaging mode over the imaging region of the object are acquired, and a panoramic image over the imaging region, a tomographic image or a three-dimensional image of an arbitrary cross section of the object is reconstructed from the image data.
